# FASCICULE DE BREVET EUROPEEN

(11) **EP 3 393 529 B1**
(45) Date de publication et mention de la délivrance du brevet: **17.03.2021**
(21) Numéro de dépôt: 16829419.7
(22) Date de dépôt: 22.12.2016
(51) Int. Cl.: A61L 2/18, A61L 2/20, B65B 55/10, B29C 49/00

(54) **PROCÉDÉ DE STÉRILISATION DES MOYENS D'ÉTIRAGE D'UN DISPOSITIF DE MOULAGE DE RÉCIPIENTS, ET INSTALLATION DE FABRICATION DE RÉCIPIENTS**
VERFAHREN ZUM STERILISIEREN DER VORRICHTUNG ZUM STRECKEN EINER BEHÄLTERFORMVORRICHTUNG UND BEHÄLTERFERTIGUNGSANLAGE
METHOD OF STERILIZING THE MEANS OF STRETCHING A CONTAINER MOULDING DEVICE AND CONTAINER MANUFACTURING PLANT

(30) Priorité: 22.12.2015 FR 1563052
(43) Date de publication de la demande: 31.10.2018
(73) Titulaire: Sidel Participations, 76930 Octeville-sur-Mer (FR)
(72) Inventeur: QUETEL, François, 76930 Octeville-sur-Mer (FR); YGER, Benjamin, 76930 Octeville-sur-Mer (FR); LETELLIER, Sandy, 76930 Octeville-sur-Mer (FR)
(74) Mandataire: Sidel Group
(86) Numéro de dépôt international: PCT/FR2016/053625
(87) Numéro de publication internationale: WO 2017/109421

(56) Documents cités:
- EP-A1- 2 772 447
- WO-A1-2006/136499
- WO-A1-2015/125808

## Description

La présente invention concerne un procédé de stérilisation des moyens d'étirage d'un dispositif de moulage de récipients, ainsi qu'une installation de fabrication de récipients.

La présente invention concerne plus particulièrement un procédé pour la stérilisation des moyens d'étirage d'un dispositif de moulage de récipients équipant une installation de fabrication de récipients en matière thermoplastique obtenus par étirage-soufflage à partir de préformes de production.

### Etat de la technique

On connaît de l'état de la technique de tels dispositifs de moulage de récipients, lesdits dispositifs de moulage équipant notamment une machine de moulage d'une installation pour la fabrication de récipients.

Dans une telle installation de fabrication de récipients, il est connu de mettre en oeuvre différentes actions dans le but de contrôler et de maîtriser la qualité microbiologique de l'environnement de fabrication comme celle des récipients.

On recherche tout particulièrement à éliminer tous les agents pathogènes, tels que des germes, spores, bactéries, etc.

En effet, de tels agents pathogènes sont susceptibles d'affecter le produit conditionné dans les récipients, notamment dans le cas de produits agro-alimentaires.

Pour ce faire, les actions ne visent pas exclusivement la décontamination des récipients ou celles des préformes de production à partir desquelles ils sont fabriqués mais également l'installation de fabrication elle-même.

Les documents de l'état de la technique cités ci-après et auxquels on se reportera pour de plus amples détails, illustrent à titre d'exemples non limitatifs de telles actions.

On peut en particulier distinguer d'une part les actions visant à détruire les agents pathogènes et, d'autre part, les actions visant plus généralement à prévenir la contamination des récipients par de tels agents.

Le document FR-2.915.127 décrit une installation de fabrication de récipients comportant une enceinte de protection délimitant une zone à l'intérieur de laquelle est agencée une machine de moulage de récipients de type souffleuse qui est alimentée par des moyens de transfert en préformes de production préalablement conditionnées thermiquement dans un four.

Selon les enseignements de ce document, l'installation comporte un système d'insufflation d'air filtré à l'intérieur de l'enceinte pour y établir notamment une surpression de manière à limiter les risques de contamination tant des préformes de production en sortie du four que des récipients fabriqués.

Le document FR-2.940.964 propose des moyens perfectionnés pour l'escamotage d'une roue de transfert intervenant entre la sortie de la machine de moulage (souffleuse) et une machine de remplissage de manière à limiter certains risques de contamination.

Ce document enseigne plus particulièrement les risques de contamination qui sont inhérents à toute intervention humaine (opérateurs) dans l'environnement immédiat des machines d'une installation de fabrication de récipients, par exemple pour de la maintenance mais d'une manière générale quel que soit le but de l'intervention.

Le document WO-03/084818 décrit par exemple un traitement de décontamination par irradiation du col de préformes de production par un rayonnement de type ultraviolet (UV), avant l'introduction desdites préformes de production dans le four.

Le document EP-2.094.312 décrit par exemple un traitement par irradiation avec un rayonnement ultraviolet (UV) mis en oeuvre de manière particulière dans un four pour décontaminer au moins la surface externe de la préforme de production en cours de conditionnement thermique.

Les documents WO-99/03667 ; WO-2006/136498 et WO 2006/136499 décrivent des exemples de traitement de décontamination de la paroi interne d'une préforme de production au moyen d'un agent stérilisant, tel que du peroxyde d'hydrogène.

Bien entendu, les différents exemples d'actions précités sont susceptibles d'être simultanément mis en oeuvre dans une même installation pour réduire de manière drastique les risques de contamination.

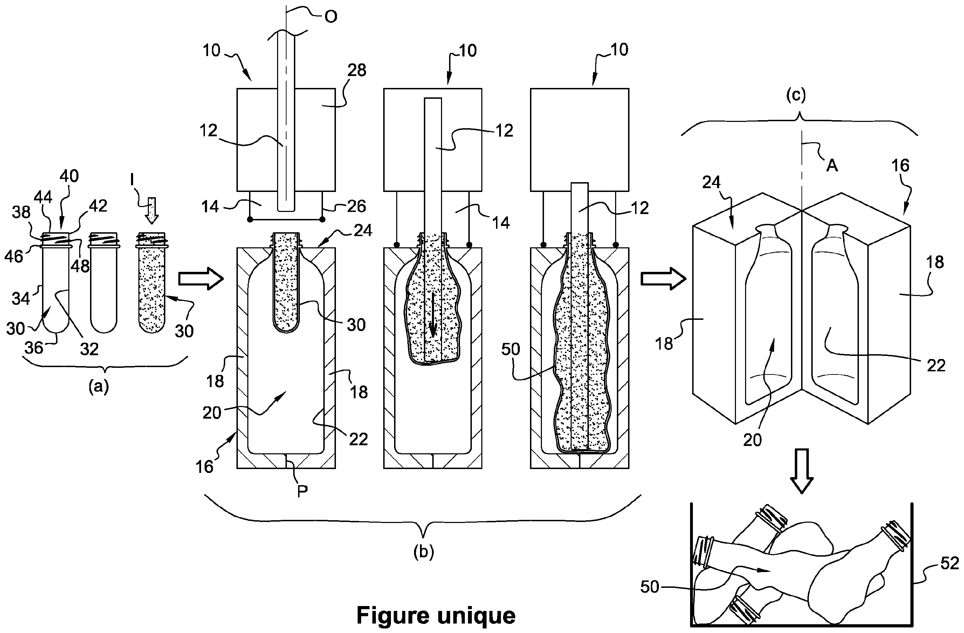

Une installation de fabrication de récipient est également décrite et représentée à la figure 1 du document EP-A1-2.292.406 auquel on se reportera pour une description détaillée.

L'invention concerne plus particulièrement une autre de ces actions qui consiste à réaliser une désinfection des moyens d'étirage de chaque dispositif de moulage de récipients au moyen d'une solution à base d'alcool.

Lors de la fabrication de récipients par étirage-soufflage, les moyens d'étirage entrent en contact avec le fond de la préforme de production pour l'étirer axialement. Il existe alors un risque de contamination de l'intérieur des récipients fabriqués par des agents pathogènes qui seraient présents sur les moyens d'étirage.

Une contamination de l'intérieur de la préforme de production par les moyens d'étirage, généralement formés par une tige d'étirage (ou d'élongation), est d'autant plus critique lorsque la décontamination de l'intérieur de ladite préforme a été réalisée en amont de la machine de moulage de récipients.

Une telle désinfection des moyens d'étirage est réalisée avant de débuter la fabrication de récipients mais cette désinfection à l'alcool ne donne toutefois pas entièrement satisfaction.

Si l'alcool présente l'avantage de pouvoir être éliminé par simple évaporation, l'alcool présente aussi l'inconvénient de n'avoir que des effets réduits à l'égard de certains agents pathogènes.

De plus, l'aspersion des moyens d'étirage avec de l'alcool est généralement réalisée par au moins un opérateur. Or, le fait pour un opérateur de pénétrer à l'intérieur de l'installation pour réaliser la désinfection des moyens d'étirage induit également des risques de contamination de l'environnement de fabrication.

Tel que décrit dans le document FR-2.915.127 précité, une installation de fabrication de récipients est généralement délimitée par une enceinte afin de pouvoir isoler l'intérieur de l'installation et d'y contrôler la qualité de l'air.

L'opération de désinfection, en exigeant la présence d'au moins un opérateur, ajoute un nouveau risque de contamination de l'environnement de fabrication par des agents pathogènes dont ledit opérateur serait porteur.

De tels risques ont par exemple déjà été notamment décrits dans le document FR-2.940.964 précité auquel on se reportera avantageusement pour de plus amples détails.

"Les documents WO2015/125808, EP2772447 et WO2006/136499 décrivent des procédés de stérilisation de préformes"

Le document US20160325482 a par ailleurs décrit une machine de soufflage et un procédé de stérilisation de cette machine. Le procédé décrit consiste à remplir la préforme avec un liquide de stérilisation et à souffler de l'air dans cette préforme pour diffuser dans la machine de formage par souffle, ledit liquide de stérilisation. Cela permet de stériliser la machine de soufflage sans intervention manuelle. Cependant cela présente notamment l'inconvénient de risquer de polluer la machine de soufflage que l'on veut stériliser par une quantité d'agent stérilisant restant dans la machine peu compatible avec les normes en vigueur pour la fabrication de récipients contenant des produits alimentaires.

Le but de la présente invention est notamment de proposer une nouvelle solution afin de réaliser une stérilisation efficace des moyens d'étirage d'un dispositif de moulage utilisé pour la fabrication de récipients en matière thermoplastique, notamment à usage alimentaire.

### Description générale de l'invention

Dans ce but, l'invention propose un procédé pour la stérilisation des moyens d'étirage d'un dispositif de moulage de récipients équipant une installation de fabrication de récipients en matière thermoplastique obtenus par étirage-soufflage à partir de préformes de production, ledit procédé de stérilisation des moyens d'étirage comporte au moins les étapes consistant à :
- introduire une quantité déterminée d'un agent stérilisant à l'intérieur d'une préforme de stérilisation,
- au cours d'une étape dite de transformation, transformer ladite préforme de stérilisation en corps creux par étirage-soufflage dans ledit dispositif de moulage.

Selon l'invention, ladite introduction de l'agent stérilisant se fait à l'état de vapeur dans une préforme de stérilisation ayant une température permettant d'obtenir un dépôt par condensation d'un film de buée uniforme sur une surface interne de ladite préforme de stérilisation, et il est procédé à la transformation successive en corps creux d'au moins deux préformes de stérilisation dans un même dispositif de moulage. De plus, l'étape de transformation consiste à obtenir un récipient partiellement formé.

Un tel procédé présente l'avantage par rapport le document US20160325482 précité que la quantité d'agent stérilisant introduit dans la machine de moulage est beaucoup plus faible. Cela permet de pouvoir beaucoup plus rapidement ramener le taux résiduel d'agent stérilisant à un niveau toléré pour la production de récipients à usage alimentaire. Cela permet ainsi de démarrer beaucoup plus vite le mode de production de récipient avec les préformes de production. En effet, la quantité d'agent stérilisant déposée dans les préformes de stérilisation par la condensation est extrêmement faible, mais suffisante pour apporter une contribution à la stérilisation des moyens d'étirage. De plus, le fait de stériliser les moyen d'étirage par l'étirage de plusieurs préformes de stérilisation successives permet de répartir l'effet stérilisant sur plusieurs opérations de transformation des préformes de stérilisation, et ainsi de réduire encore la quantité d'agent stérilisant entrant dans la machine de moulage. Finalement, la quantité d'agent stérilisant introduite dans la machine de moulage est sans commune mesure avec celle impliquée par le remplissage des préformes par un agent stérilisant liquide.

Le procédé de stérilisation des moyens d'étirage selon l'invention permet également de supprimer la désinfection à l'alcool des moyens d'étirage opérée manuellement par au moins un opérateur, avant la fabrication de récipients dans l'installation, dans la mesure où c'est l'agent stérilisant contenu dans la préforme de stérilisation qui agit pour stériliser les moyens d'étirage lorsque ces derniers pénètrent dans la préforme de stérilisation.

Avantageusement, le procédé de stérilisation selon l'invention est mis en oeuvre automatiquement dans une installation de fabrication de récipients comportant une machine de moulage équipée d'au moins un dispositif de moulage pourvu de moyens d'étirage.

La présence d'un opérateur à l'intérieur de l'installation pour une intervention manuelle n'est donc plus nécessaire, le procédé de stérilisation selon l'invention permet ainsi de supprimer totalement les risques de contamination de l'environnement de fabrication associés.

Grâce à la stérilisation des moyens d'étirage, les risques de contamination des récipients et tout particulièrement les risques de contamination liés à la présence d'agents pathogènes sur les moyens d'étirage d'un dispositif de moulage sont encore plus réduits voire supprimés.

Selon un mode avantageux de réalisation, l'agent stérilisant contient du peroxyde d'hydrogène, le procédé comprenant une étape d'activation du peroxyde d'hydrogène de façon à former des radicaux libres, ladite activation de l'agent stérilisant de chaque préforme de stérilisation ayant lieu avant et/ou pendant l'étape de transformation de ladite préforme de stérilisation en corps creux par étirage-soufflage.

Dans le mode de réalisation ci-dessus, l'activation du peroxyde d'hydrogène permet d'amener des radicaux libres extrêmement germicides directement sur les moyens d'étirage. Cela confère une efficacité de destruction des micro-organismes très suffisante malgré la faible quantité d'agent stérilisant introduit dans la machine de soufflage. En effet le pouvoir germicide du peroxyde d'hydrogène activé est sans commune mesure avec celui obtenu avec un agent stérilisant liquide, notamment une désinfection à l'alcool.

Le procédé de stérilisation selon l'invention est susceptible d'être mis en oeuvre dans une installation existante, en utilisant un dispositif de décontamination existant mais pour stériliser les moyens d'étirage avec l'agent stérilisant introduit dans au moins une préforme de stérilisation au cours du mode de fonctionnement, dit mode de stérilisation, de l'installation.

Avantageusement, la série de corps creux évacués après la stérilisation des moyens d'étirage est éliminée et recyclée.

Selon d'autres caractéristiques de l'invention pouvant être utilisée séparément ou en combinaison :
- le procédé comprend une étape de conditionnement thermique des préformes de production et des préformes (de stérilisation, l'étape d'introduction d'une quantité d'agent stérilisant se faisant avant et/ou pendant l'étape de conditionnement thermiques des préformes de stérilisations,
- le procédé comprend une étape consistant à évacuer hors de l'installation, à l'issue de l'étape de transformation, les corps creux obtenus à partir des préformes de stérilisation. Cela permet de bien séparer le mode de stérilisation du mode de production. L'évacuation permet de réduire, voire d'éliminer le risque de recontamination de machine de soufflage, ou des machine de traitement ultérieur des récipients.

- l'étape de transformation consiste à obtenir un récipient partiellement formé ;
- l'étape de transformation consiste à obtenir un récipient entièrement formé ;
- lesdites étapes du procédé de stérilisation des moyens d'étirage sont mises en œuvre dans un mode de fonctionnement de l'installation, dit mode de stérilisation, dans lequel le dispositif de moulage fabrique une série de corps creux ;
- les moyens d'étirage sont maintenus à l'intérieur de la préforme de stérilisation comportant l'agent stérilisant pendant une durée donnée qui est supérieure ou égale à celle d'une préforme de production ;
- ladite durée donnée de contact des moyens d'étirage avec l'agent stérilisant contenu à l'intérieur de la préforme de stérilisation est obtenue en contrôlant sélectivement des moyens d'actionnement qui commandent en déplacement lesdits moyens d'étirage ;
- ladite durée donnée de contact des moyens d'étirage avec l'agent stérilisant contenu à l'intérieur de la préforme de stérilisation est obtenue en agissant sur une vitesse d'entrainement en rotation d'un carrousel d'une machine de moulage de l'installation de fabrication comportant le dispositif de moulage de récipients ;
- ledit agent stérilisant introduit dans une préforme de stérilisation est le même agent stérilisant que celui utilisé pour une préforme de production ;
- la quantité déterminée d'agent stérilisant introduite dans la préforme de stérilisation est supérieure ou égale à celle introduite dans une préforme de production ;
- ledit agent stérilisant introduit dans la préforme de stérilisation présente des paramètres physico-chimiques différents de celui introduit dans une préforme de production ;
- ledit agent stérilisant introduit dans la préforme de stérilisation présente une concentration supérieure à celui introduit dans une préforme de production ;
- la préforme de stérilisation est chauffée, avant l'étape de transformation en corps creux, à une température donnée qui est différente de celle d'une préforme de production ;
- la préforme de stérilisation est moulée par étirage-soufflage au moyen d'un fluide de soufflage présentant une pression maximale qui est inférieure ou égale à celle la pression finale de soufflage d'une préforme de production ;
- la préforme de stérilisation est identique à une préforme de production.

Selon un autre aspect, l'invention porte également sur une installation de fabrication de récipients en matière thermoplastique, pour la mise en œuvre du procédé précité, présentant un mode de stérilisation et un mode de production, l'installation comprenant :
- une machine de moulage équipée d'au moins un dispositif de moulage ayant des moyens d'étirage, le dispositif de moulage étant conçu pour fabriquer par étirage-soufflage lesdits récipients à partir de préformes de production lorsque l'installation est en mode de production,
- des moyens de transfert pour transporter, lorsque l'installation est en mode de production, lesdits récipients fabriqués entre la machine de moulage et une autre machine située en aval,
- un dispositif de décontamination, actionné au moins lorsque l'installation est en mode de stérilisation, et conçu pour introduire de l'agent stérilisant dans au moins une préforme de stérilisation, la machine de moulage transformant la préforme de stérilisation en corps creux étiré par lesdits moyens d'étirage.

Avantageusement, l'installation comprend des moyens d'évacuation actionnable en mode de stérilisation pour évacuer les corps creux hors des moyens de transfert en amont de ladite autre machine.

Selon d'autres caractéristiques de l'invention pouvant être utilisée séparément ou en combinaison :
- l'installation comprend un four de conditionnement thermique des préformes de production et/ou des préformes de stérilisation, le dispositif de décontamination étant disposé en amont, à l'intérieur ou en aval du four de conditionnement thermique.
- le dispositif de décontamination est conçu pour introduire l'agent stérilisant à l'état de vapeur dans une préforme de stérilisation ayant une température permettant d'obtenir un dépôt par condensation d'un film de buée uniforme sur une surface interne de ladite préforme de stérilisation.
- le mode de stérilisation procède à la transformation successive d'au moins en corps creux d'au moins deux préformes de stérilisation dans un même dispositif de moulage.
- l'installation comprend un moyen de collecte des corps creux évacués en mode de stérilisation.
- l'installation comprend au moins trois machines de traitement de récipients reliées entre elles par des dispositifs de convoyage convoyant individuellement chaque récipient de sorte que, lorsque l'installation est en mode de production, les machines de traitement traitent l'une après l'autre chaque récipient d'une succession de récipients ; ladite machine de moulage étant une première machine de traitement de récipients, ladite autre machine étant une deuxième machine de traitement de récipients directement reliée en aval de la première machine de traitement par lesdits moyens de transfert, et une boucheuse conçue pour isoler de manière aseptique l'intérieur d'un récipient par rapport à l'environnement extérieur constituant une troisième machine de traitement.

D'autres caractéristiques et avantages de l'invention apparaîtront à la lecture de la description qui va suivre pour la compréhension de laquelle on se reportera à la figure unique illustrant les étapes du procédé de stérilisation des moyens d'étirage selon l'invention.

On a représenté schématiquement sur la figure unique la mise en œuvre des étapes du procédé pour la stérilisation des moyens d'étirage d'un dispositif 10 de moulage de récipients en matière thermoplastique obtenus par étirage-soufflage à partir de préformes de production.

### Description détaillée de l'invention

Selon l'exemple de réalisation illustré sur la figure, le dispositif 10 de moulage représenté est notamment apte à fabriquer par étirage-soufflage des récipients en matière thermoplastique à partir de préformes de production.

Une telle fabrication de récipients correspond à un mode de fonctionnement, dit mode de production, de l'installation dans lequel le dispositif 10 de moulage est utilisé pour la fabrication de récipients à partir de préformes de production.

Les récipients en matière thermoplastique fabriqués grâce à un tel dispositif 10 de moulage sont par exemple des bouteilles, des flacons ou des pots, notamment mais non exclusivement destinés à l'industrie agro-alimentaire.

On décrira ci-après le dispositif 10 de moulage selon l'exemple de réalisation représenté sur la figure.

Le dispositif 10 de moulage comporte au moins des moyens 12 d'étirage et des moyens 14 de soufflage.

Le dispositif 10 de moulage comporte un moule 16 comportant au moins deux demi-moules 18 qui sont montés mobiles entre au moins une position ouverte et une position fermée.

Les moyens 12 d'étirage et les moyens 14 de soufflage sont agencés axialement au-dessus du moule 16.

Dans l'exemple de réalisation, le dispositif 10 de moulage est un dispositif de moulage du type "en portefeuille" (ou *"book like opening"* en anglais).

Le dispositif 10 de moulage comporte au moins deux demi-moules 18 qui sont montés mobiles autour d'un axe A commun de rotation, respectivement entre lesdites positions ouverte et fermée.

Le mouvement d'ouverture ou de fermeture des deux demi-moules 18, respectivement entre lesdites positions ouverte et fermée, est par exemple commandé par des moyens mécaniques de type à galet et à came. Le mouvement d'ouverture ou de fermeture des demi-moules 18 est notamment fonction de leur position angulaire.

En variante, le mouvement d'ouverture ou de fermeture des demi-moules 18 est commandé par des moyens électriques tels que des moteurs électriques positionnés sur l'axe du moule. La commande de l'ouverture ou de la fermeture des demi-moules 18 est dès lors susceptible d'être commandée indépendamment de la position angulaire du moule du dispositif de moulage.

Un tel dispositif 10 de moulage équipe généralement une machine de moulage ou "souffleuse" de type rotative (non représentée).

De préférence, une telle machine de moulage comporte des dispositifs 10 de moulage qui sont répartis de manière régulière en périphérie d'un carrousel, lequel carrousel est entraîné en rotation autour d'un axe central.

En variante, il est également connu d'utiliser pour la fabrication de récipients un dispositif 10 de moulage dont les demi-moules 18 sont montés mobiles en translation entre au moins une position ouverte et une position fermée.

Les demi-moules 18 délimitent ensemble une cavité 20 de moulage lorsque, en position fermée, lesdits demi-moules 18 sont réunis selon un plan P de joint. La cavité 20 de moulage est délimitée par une empreinte 22 de moulage que comporte chaque demi-moule 18 et qui correspond en creux au récipient à fabriquer.

En variante non représentée, le moule 16 est réalisé en trois parties, respectivement deux demi-moules 18 et un fond de moule qui comporte une empreinte correspondant au fond du récipient à fabriquer, par exemple un fond de forme pétaloïde.

Les moyens 14 de soufflage sont montés mobiles axialement entre au moins une position haute et une position basse dans laquelle lesdits moyens 14 de soufflage coopèrent de manière étanche avec une face 24 supérieure du moule 16 occupant la position fermée.

Les moyens 14 de soufflage comportent notamment des moyens 26 d'étanchéité destinés à coopérer avec ladite face 24 supérieure du moule 16 lorsqu'au moins un fluide sous pression, généralement de l'air, est introduit par les moyens 14 de soufflage à l'intérieur d'une préforme de production pour fabriquer le récipient par étirage-soufflage.

De tels moyens 14 de soufflage sont connus de l'état de la technique et ne seront par conséquent pas décrits plus en détails. A titre d'exemples non limitatifs, on se pourra toutefois se reporter aux documents FR-2.764.544 ou FR-2.912.678.

Dans l'exemple de réalisation illustré sur la figure, les moyens 12 d'étirage sont constitués par une tige.

Les moyens 12 d'étirage sont montés mobiles axialement suivant un axe O, respectivement entre au moins une position escamotée et une position d'utilisation dans laquelle lesdits moyens 12 d'étirage sont aptes à réaliser un étirage.

Le dispositif 10 de moulage comporte des moyens 28 d'actionnement des moyens 12 d'étirage qui sont commandés pour déplacer axialement lesdits moyens 12 d'étirage entre au moins lesdites positions escamotée et d'utilisation.

De préférence, les moyens 28 d'actionnement sont formés par un moteur électrique permettant de commander sélectivement le déplacement axial des moyens 12 d'étirage.

En variante, les moyens 28 d'actionnement sont constitués par des moyens mécaniques du type à galet et à came qui commandent le déplacement axial des moyens 12 d'étirage en fonction notamment de la position angulaire du dispositif 10 de moulage lorsque ledit dispositif 10 de moulage se déplace en rotation avec le carrousel d'une machine de moulage.

Les préformes de production sont généralement obtenues par moulage par injection de matière thermoplastique comme le PET (*PolyEthylene Terephtalate*).

De telles préformes préalablement fabriquées doivent ultérieurement être conditionnées thermiquement afin d'en ramollir la matière constitutive et permettre leur transformation en récipients par soufflage ou par étirage-soufflage.

En variante, il est également connu de transformer directement en récipient une préforme de production obtenue par extrusion, un tel procédé de fabrication de récipients étant généralement appelé "extrusion-soufflage".

Dans un cas comme dans l'autre, le récipient est obtenu à partir d'une préforme de production chaude qui subit des opérations d'étirage et de soufflage dans la cavité 20 du moule 16 du dispositif 10 de moulage en position fermée.

La machine de moulage (ou "souffleuse") comportant au moins un dispositif 10 de moulage de récipients constitue généralement l'une des unités d'une installation (non représentée) de fabrication de récipients.

Une installation de fabrication de récipients comporte par exemple au moins un four pour le conditionnement thermique des préformes de production. Le four de conditionnement thermique constitue une autre unité de l'installation.

L'installation comporte des moyens de transfert, tels que des roues munies parfois de pinces, agencés entre les différentes unités pour transporter des préformes ou des récipients, par exemple pour transporter des préformes de production depuis la sortie du four jusqu'à la machine de moulage située en aval.

Des moyens de transfert sont également utilisés dans l'installation pour transporter les récipients fabriqués entre la machine de moulage et au moins une autre machine située en aval telle qu'une remplisseuse, une boucheuse, etc.

Avantageusement, les moyens de transfert de l'installation sont notamment synchronisés entre eux de manière automatique et peuvent être désynchronisés à la demande, ce qui, dans certains cas, s'avère particulièrement utile lorsque les installations sont rotatives : il est en effet possible, en associant lesdits moyens de transfert à une commande pilotée de l'ouverture des moules, de prolonger le temps de maintien du corps creux dans le moule en faisant rester le corps creux au-delà d'une révolution du carrousel tout en maintenant les moyens d'étirage en position d'étirage des préformes. En d'autres termes, l'évacuation des corps creux peut être retardée.

Selon les applications, l'installation comporte un dispositif de décontamination (non représenté) pour stériliser au moins l'intérieur des préformes de production destinées à être transformées en récipients ou obtenir des récipients stériles.

L'installation peut encore comporter en aval de la machine de moulage une autre unité comportant au moins une machine de remplissage dans laquelle on procède au remplissage des récipients fabriqués.

De préférence, on réalise également dans une telle unité au moins la fermeture desdits récipients après leur remplissage, la fermeture s'effectuant par exemple par bouchage.

Tel qu'expliqué en préambule de la description, on recherche encore et toujours à améliorer la qualité de fabrication des récipients en luttant contre les risques de contamination microbiologiques par des agents pathogènes.

L'invention a pour but de réduire encore ces risques de contamination en procédant à une stérilisation des moyens 12 d'étirage d'un dispositif 10 de moulage tel que celui décrit précédemment en référence à la figure.

Les moyens d'étirage à stériliser selon le procédé sont les moyens 12 d'étirage d'un dispositif 10 de moulage de récipients équipant une installation de fabrication de récipients en matière thermoplastique obtenus par étirage-soufflage à partir de préformes de production.

Une telle installation précédemment décrite est par exemple représentée sur la figure 5 du document WO-99/03667 ou encore sur les figures 1 et 4 des documents WO-2006/136498 et WO-2006/136499.

Dans l'exemple de réalisation, le dispositif 10 de moulage équipe plus précisément une machine de moulage (ou souffleuse), ladite machine de moulage formant l'une des unités de l'installation de fabrication de récipients en matière thermoplastique.

Conformément à l'invention, le procédé de stérilisation des moyens 12 d'étirage d'un dispositif 10 de moulage de récipients, équipant une installation de fabrication de récipients en matière thermoplastique par étirage-soufflage de préformes de production, comporte au moins les étapes consistant à :
- introduire une quantité déterminée d'un agent stérilisant à l'intérieur d'une préforme 30 de stérilisation,
- lors d'une étape dite de transformation, transformer ladite préforme 30 de stérilisation en corps creux 50 par étirage-soufflage dans ledit dispositif 10 de moulage, et
- évacuer hors de l'installation, à l'issue de l'étape de transformation, le corps creux 50 obtenu lors de cette étape à partir de la préforme 30 de stérilisation.

Les étapes du procédé de stérilisation des moyens 12 d'étirage sont mises en œuvre dans un mode de fonctionnement de l'installation, dit mode de stérilisation, dans lequel le dispositif 10 de moulage fabrique une série de corps creux à partir des préformes 30 de stérilisation.

Avantageusement, le procédé de stérilisation des moyens 12 d'étirage est au moins mis en œuvre avant de réaliser une fabrication de récipients à partir de préforme de production.

De préférence, les étapes du procédé de stérilisation des moyens 12 d'étirage sont effectuées lors d'une phase de démarrage de l'installation préparatoire à la fabrication de récipients.

Dans l'installation, le mode de fonctionnement, dit mode de stérilisation, est utilisé préalablement à l'autre mode de fonctionnement, dit mode de production, au cours duquel des récipients sont fabriqués par au moins un dispositif 10 de moulage à partir de préformes de production.

Dans le mode de stérilisation, on utilise des préformes 30 de stérilisation destinées à être transformées en corps creux tandis que dans le mode de production on utilise des préformes de production destinées à être transformées en récipients.

Dans l'exemple de réalisation et tel que représenté sur la figure, la préforme 30 de stérilisation est avantageusement identique à une préforme de production.

La préforme 30 de stérilisation comporte une surface 32 interne délimitée par un corps 34 fermé à une extrémité par un fond 36 et qui, à l'autre extrémité, comporte un col 38 délimitant une ouverture 40 d'accès à l'intérieur de la préforme 30.

Le col 38 de la préforme 30 de stérilisation présente sa forme définitive à l'issue de sa fabrication réalisée de préférence par moulage par injection.

La préforme 30 de stérilisation étant identique à une préforme de production, le col 38 de la préforme 30 de stérilisation comporte un filetage 42 normalement destiné à permettre la fermeture du récipient final par un bouchon à vis complémentaire.

L'ouverture 40 est délimitée par un bord 44 (ou buvant) du col 38, ladite ouverture 40 constituant le seul accès à l'intérieur de la préforme 30 puisque le corps 34 est fermé axialement à l'opposé par le fond 36.

Dans l'exemple représenté sur la figure, le col 38 comporte une collerette 46 qui s'étend radialement en saillie vers l'extérieur. Le col 38 comporte encore ici une gorge 48 annulaire qui est adjacente à ladite collerette 46.

Toutefois, il doit être bien compris que la préforme 30 de stérilisation pourrait également être différente d'une préforme de production destinée à la fabrication d'un récipient.

En effet, la préforme de production présente des caractéristiques (dimensions, répartition de la matière, etc.) qui sont déterminées en fonction du récipient final à obtenir, en particulier sa forme ou encore sa contenance.

Par comparaison, la préforme 30 de stérilisation est uniquement destinée à stériliser les moyens 12 d'étirage grâce à l'agent stérilisant qui y est introduit lors de la première étape du procédé de stérilisation selon l'invention.

Par comparaison avec une préforme de fabrication, la préforme 30 de stérilisation est destinée à être transformée en un corps creux 50 qui est évacué de l'installation après la stérilisation des moyens 12 d'étirage.

Une préforme 30 de stérilisation pourrait ainsi présenter un col 38 différent, ledit col 38 ne comportant notamment pas de filetage 42 et/ou de gorge 48 dès lors que le corps creux 50 est évacué hors de l'installation après son extraction du dispositif 10 de moulage.

Le corps creux 50 obtenu à partir de la préforme 30 de stérilisation est évacué en amont de l'unité de remplissage lorsque l'installation comporte une telle unité. Contrairement au récipient, le corps creux 50 n'est en effet pas destiné à être rempli, ni fermé par bouchage par exemple au moyens d'un bouchon à vis.

Outre le col 38, la préforme 30 de stérilisation pourrait également se distinguer d'une préforme de production par son poids.

De préférence, la préforme 30 de stérilisation est alors plus légère qu'une préforme de production afin de réaliser des économies de coût par rapport à la quantité de matière thermoplastique.

La préforme 30 de stérilisation comporte moins de matière thermoplastique, par exemple un corps 34 de moindre épaisseur, puisque la finalité n'est pas d'obtenir un récipient mais de stériliser les moyens 12 d'étirage.

Dans l'exemple illustré par la figure, le corps creux 50 obtenu à l'issue de l'étape de transformation de la préforme 30 de stérilisation en corps creux 50 est constitué par un récipient partiellement formé.

En variante non représentée, le corps creux 50 est un récipient. Lorsque la préforme 30 de stérilisation est identique à une préforme de production, le corps creux 50 est susceptible d'être identique à un récipient obtenu par moulage à partir d'une préforme de production.

Par comparaison, on comprendra cependant qu'un corps creux 50 constitué par un récipient partiellement formé permet de réaliser certaines économies, notamment en utilisant moins d'air pour le soufflage de la préforme 30 de stérilisation.

Avantageusement, le corps creux 50 constitué par un récipient partiellement formé présente un moindre volume qu'un récipient de sorte que la stérilisation des moyens 12 d'étirage par l'agent stérilisant présent à l'intérieur de la préforme 30 de stérilisation est facilitée.

Le procédé de stérilisation des moyens 12 d'étirage selon l'invention remplace avantageusement la désinfection à l'alcool des moyens 12 d'étirage réalisée jusqu'alors manuellement par au moins un opérateur avant la fabrication de récipients dans l'installation.

Par comparaison avec la désinfection à l'alcool, le procédé de stérilisation permet d'obtenir des degrés de décontamination bien supérieur et pouvant par exemple atteindre "6Log" selon l'agent stérilisant utilisé.

De préférence, l'agent stérilisant est du peroxyde d'hydrogène (H₂O₂). En variante, l'agent stérilisant est par exemple constitué par de l'hydroxyde de sodium (NaOH).

On rappelle que la quantité de micro-organismes est susceptible d'être dénombrée par comptage après notamment des opérations de lavage, de filtration et de mise en culture. On détermine ainsi une réduction logarithmique du nombre de micro-organismes par exemple dite de l'ordre de 3Log (ou encore 3D) équivalent à 1000 unités (10³).

Avantageusement, le procédé de stérilisation selon l'invention est mis en œuvre automatiquement dans l'installation de fabrication de récipients lorsque l'installation est en mode de fonctionnement, dit mode de stérilisation.

Par comparaison avec la désinfection à l'alcool, le procédé de stérilisation ne nécessite plus l'intervention d'un opérateur à l'intérieur de l'installation délimitée par son enceinte, grâce à quoi les risques de contamination de l'environnement de fabrication associés à la présence de l'opérateur sont supprimés.

La stérilisation chimique des moyens 12 d'étirage au moyen d'un agent stérilisant, tel que le peroxyde d'hydrogène (H₂O₂), permet à tout le moins de réduire, voire de supprimer, les agents pathogènes susceptibles d'être présents sur les moyens 12 d'étirage du dispositif 10 de moulage.

Grâce à la stérilisation selon l'invention, on supprime les risques de contamination résultant du contact des moyens 12 d'étirage avec l'intérieur des préformes de production à partir desquelles les récipients sont fabriqués par le dispositif 10 de moulage.

Tel qu'illustré sur la figure, le procédé de stérilisation des moyens 12 d'étirage comporte au moins une étape (a) consistant à introduire une quantité déterminée d'un agent stérilisant à l'intérieur d'une préforme 30 de stérilisation.

On a représenté schématiquement par une flèche référencée "I" l'introduction de la quantité déterminée d'agent stérilisant à l'intérieur de la préforme 30 de stérilisation.

Sur la figure, la présence d'agent stérilisant dans la préforme 30 de stérilisation puis le corps creux 50 a été matérialisée par des points dont le choix relève d'une simple convention sans référence aucune notamment à l'état de l'agent stérilisant.

De préférence, l'introduction de l'agent stérilisant selon l'étape (a) est réalisée dans un flux de préformes 30 de stérilisation, par exemple successivement dans plusieurs préformes 30 de stérilisation représentées sur la figure. En effet, pour atteindre le degré de stérilisation requis, il peut être nécessaire d'appliquer le procédé de stérilisation sur plusieurs préformes de stérilisation successives, de façon à obtenir l'effet germicide requis pour les moyens 12 d'étirage.

L'agent stérilisant introduit dans la ou les préformes 30 de stérilisation peut être à l'état liquide et/ou gazeux, notamment à l'état de vapeur.

Lorsque l'installation est en mode de fonctionnement, dit de production, et comporte un dispositif de décontamination de préformes (non représenté), le dispositif de décontamination l'équipant est utilisé pour introduire un agent stérilisant à l'intérieur des préformes de production.

Dans le mode de stérilisation de l'installation, l'agent stérilisant est de préférence introduit dans une préforme 30 de stérilisation par ce même dispositif de décontamination que comporte l'installation.

Avantageusement, ledit agent stérilisant introduit dans une préforme 30 de stérilisation est le même agent stérilisant que celui utilisé pour une préforme de production.

Selon le type de dispositif de décontamination, l'agent stérilisant est introduit dans une préforme 30 de stérilisation qui est dite "froide" ou "chaude".

Par préforme 30 de stérilisation "froide", on désigne notamment une préforme à température ambiante dans laquelle l'agent stérilisant est introduit avant de procéder au conditionnement thermique de la préforme 30 de stérilisation dans un four.

Le conditionnement thermique d'une préforme 30 de stérilisation est notamment destiné à permettre d'étirer axialement la préforme 30 de stérilisation avec les moyens 12 d'étirage du dispositif 10 de moulage lors d'une étape (b) dite de transformation, qui sera décrite ultérieurement.

Le conditionnement thermique de la préforme 30 de stérilisation a également pour effet d'activer thermiquement l'agent stérilisant préalablement introduit.

Selon un premier exemple de réalisation de l'étape (a), l'agent stérilisant est introduit à l'état liquide à l'intérieur de la préforme 30 de stérilisation froide et la préforme 30 de stérilisation est ensuite chauffée.

Dans le cas d'un agent stérilisant formé par du peroxyde d'hydrogène (H₂O₂), le chauffage opéré va activer thermiquement l'agent et permettre d'obtenir l'effet stérilisant.

De préférence, le chauffage est réalisé par rayonnement dans un four de conditionnement thermique. En variante, le chauffage est réalisé avec de l'air chaud ou tout autre moyen équivalent permettant d'élever la température de la préforme.

De préférence, l'agent stérilisant à l'état liquide est pulvérisé à l'intérieur de la préforme 30 de stérilisation. En variante, la préforme 30 de stérilisation est remplie d'agent stérilisant à l'état liquide puis vidée.

La mise en œuvre du procédé de stérilisation des moyens 12 d'étirage selon ce premier exemple est notamment susceptible d'être réalisé avec à un dispositif de décontamination selon les enseignements du document WO-99/03667 précité.

Dans un tel exemple, la stérilisation des moyens 12 d'étirage est obtenue lors de l'étape (b) de transformation lorsque lesdits moyens 12 d'étirage entrent en contact avec l'agent stérilisant présent à l'intérieur de la préforme 30 de stérilisation.

L'agent stérilisant présent à l'intérieur de la préforme 30 de stérilisation est notamment à l'état gazeux suite au chauffage qui provoque l'évaporation de tout ou partie de l'agent stérilisant.

Selon un deuxième exemple de réalisation de l'étape (a), l'agent stérilisant est introduit dans une préforme 30 de stérilisation "froide" à l'état de vapeur de manière à obtenir le dépôt par condensation d'un film de buée uniforme sur la surface 32 interne de ladite préforme 30.

La préforme 30 de stérilisation est ensuite chauffée par rayonnement dans un four de l'installation.

La mise en œuvre du procédé de stérilisation des moyens 12 d'étirage est susceptible d'être réalisé avec à un dispositif de décontamination selon les enseignements techniques des documents WO-2006/136498 ou WO-2006/136498 précités.

Par préforme 30 de stérilisation "chaude", on désigne notamment une préforme chaude présentant une température déterminée qui résulte d'un chauffage, en variante de sa fabrication par extrusion, et dans laquelle l'agent stérilisant est introduit.

Une telle préforme chaude est par exemple obtenue après avoir procédé au conditionnement thermique de la préforme 30 de stérilisation dans un four pour en permettre axialement l'étirage par les moyens 12 d'étirage du dispositif 10 de moulage lors de l'étape (b) de transformation qui sera décrite plus en détail ultérieurement.

Selon un troisième exemple de réalisation de l'étape (a), l'agent stérilisant est introduit à l'état gazeux à l'intérieur d'une préforme 30 de stérilisation qui est chaude.

De préférence, l'introduction de l'agent stérilisant est réalisée lors du transfert des préformes 30 de stérilisation entre le four de conditionnement de l'installation et la machine de moulage comportant au moins un dispositif 10 de moulage muni de moyens 12 d'étirage.

Ainsi qu'on l'aura compris au moyen des trois exemples de réalisation qui viennent d'être décrits, l'étape (a) est avantageusement réalisée avec le dispositif de décontamination de l'installation de fabrication de récipients que comporte le dispositif 10 de moulage.

Un tel dispositif de décontamination est alors utilisé dans le mode de stérilisation de l'installation pour réaliser l'étape (a), ce qui en constitue une nouvelle utilisation dans un mode de fonctionnement particulier.

En effet, le dispositif de décontamination était jusqu'alors exclusivement utilisé dans le mode de production de l'installation et pour décontaminer l'intérieur de préformes de production destinées à être transformées en récipients.

En variante, la quantité déterminée d'agent stérilisant est introduite dans la préforme 30 de stérilisation lors de l'étape (a) par des moyens supplémentaires qui sont distincts de ceux du dispositif de décontamination utilisé pour décontaminer l'intérieur des préformes de production.

L'utilisation d'un dispositif de décontamination, tel que par exemple l'un de ceux décrits dans les documents précités pour les trois exemples de réalisation, est avantageusement réalisée avec des paramètres de fonctionnement qui sont déterminés et propres au mode de fonctionnement de production de l'installation comportant le dispositif 10 de moulage.

En effet, en mode de stérilisation de l'installation, le dispositif 10 de moulage est utilisé pour obtenir, à partir de préformes 30 de stérilisation, une série de corps creux 50 qui sont destinés à être évacués de l'installation après la stérilisation des moyens 12 d'étirage.

Avantageusement, la quantité déterminée d'agent stérilisant introduite dans une préforme 30 de stérilisation est donc susceptible d'être différente de celle introduite dans une préforme de production.

De préférence, la quantité déterminée d'agent stérilisant introduite dans une préforme 30 de stérilisation est supérieure ou égale à la quantité d'agent stérilisant introduite dans une préforme de production.

En effet et contrairement à un récipient, la quantité résiduelle d'agent stérilisant présente dans un corps creux 50 importe peu dès lors que le corps creux 50 est éliminé.

Avantageusement, ledit agent stérilisant introduit dans la préforme 30 de stérilisation présente des paramètres physico-chimiques différents des paramètres physico-chimiques de l'agent stérilisant introduit dans une préforme de production.

De préférence, l'agent stérilisant introduit dans la préforme 30 de stérilisation présente une concentration supérieure à la concentration de l'agent stérilisant introduit dans une préforme de production.

La préforme 30 de stérilisation a pour fonction de contenir l'agent stérilisant utilisé pour la stérilisation des moyens 12 d'étirage du dispositif 10 de moulage.

De préférence, la préforme 30 de stérilisation est chauffée, avant l'étape (b) de transformation, à une température donnée qui est différente de la température de moulage d'une préforme de production.

De préférence, la préforme 30 de stérilisation est chauffée, avant l'étape (b) de transformation, à une température donnée qui est supérieure ou égale à la température de moulage d'une préforme de production.

Avantageusement, la valeur de la température à laquelle est chauffée la préforme 30 de stérilisation est déterminée en fonction de la quantité déterminée d'agent stérilisant introduit avant le chauffage à l'intérieur de la préforme 30 de stérilisation.

La valeur de la température est alors notamment déterminée pour obtenir l'activation thermique de l'agent stérilisant, le changement d'état par évaporation de tout ou partie de l'agent stérilisant.

En variante, la préforme 30 de stérilisation est chauffée, avant l'étape (b) de transformation, à une température donnée qui est inférieure ou égale à la température de moulage d'une préforme de production.

La valeur de la température est alors notamment déterminée pour permettre, lors de ladite étape (b), l'étirage axial de la préforme 30 de stérilisation par les moyens 12 d'étirage stérilisés.

La préforme 30 de stérilisation est destinée à la stérilisation des moyens 12 d'étirage. Aussi, par comparaison avec une préforme de production, l'obtention d'un récipient bien façonné n'est pas nécessairement recherchée lors de l'étape de transformation.

C'est la raison pour laquelle, le corps creux 50 obtenu par étirage-soufflage à partir de la préforme 30 de stérilisation peut notamment être un récipient partiellement formé, tel qu'un récipient intermédiaire, comme cela est illustré sur la figure.

Tel qu'illustré sur la figure et indiqué précédemment, le procédé de stérilisation des moyens 12 d'étirage selon l'invention comporte une étape (b) consistant à transformer ladite préforme 30 de stérilisation en corps creux 50 par étirage-soufflage dans ledit dispositif 10 de moulage.

Après l'étape (a) au cours de laquelle l'agent stérilisant est introduit à l'intérieur d'une préforme 30 de stérilisation chaude, ladite préforme 30 de stérilisation est transférée jusqu'au dispositif 10 de moulage.

La préforme 30 de stérilisation contenant l'agent stérilisant (matérialisé par des points) est introduite dans le moule 16 du dispositif 10 de moulage en position ouverte, les demi-moules 18 du moule 16 sont ensuite déplacés vers la position fermée.

Tel qu'illustré sur la première vue (à gauche) de la figure pour l'étape (b), la préforme 30 de stérilisation est maintenue dans le moule 16 en position fermée par l'intermédiaire de sa collerette 46 qui est en appui sur la face 24 supérieure.

La préforme 30 de stérilisation s'étend axialement à travers une ouverture du moule 16 ménagée dans la face 24 supérieure, le col 38 de la préforme 30 s'étendant en saillie hors du moule 16 et le corps 34 s'étendant à l'intérieur de la cavité 20 de moulage.

Tel qu'illustré sur la première vue (à gauche) de la figure pour l'étape (b), les moyens 12 d'étirage sont en position escamotée et les moyens 14 de soufflage en position haute.

Ensuite et tel qu'illustré sur la deuxième vue (au centre) de la figure pour l'étape (b), les moyens 14 de soufflage sont déplacés axialement de position haute vers la position basse dans laquelle les moyens 26 d'étanchéité coopèrent avec la face 24 supérieure du moule 16.

Les moyens 14 de soufflage procèdent à l'introduction d'au moins un fluide de soufflage, tel que de l'air sous pression, à l'intérieur de la préforme 30 de stérilisation pour réaliser l'étape de transformation par étirage-soufflage de la préforme 30 de stérilisation à l'issue de laquelle le corps creux 50 est obtenu.

Avantageusement, la préforme 30 de stérilisation est transformée par étirage-soufflage au moyen d'un fluide de soufflage présentant une pression maximale qui est différente de la pression finale de soufflage d'une préforme de production.

De préférence, la préforme 30 de stérilisation est transformée par étirage-soufflage au moyen d'un fluide de soufflage présentant une pression maximale qui est inférieure à la pression finale de soufflage d'une préforme de production.

C'est la raison pour laquelle, le corps creux 50 obtenu et illustré sur la troisième vue (à droite) de la figure pour l'étape (b) peut être un récipient partiellement formé.

En variante non représentée, la préforme 30 de stérilisation est transformée par étirage-soufflage au moyen d'un fluide de soufflage présentant une pression maximale qui est égale à la pression finale de soufflage d'une préforme de production.

Dans une telle variante, le corps creux 50 obtenu à partir de la préforme 30 de stérilisation est susceptible d'être totalement moulé et de constituer un récipient à la forme de la cavité 20 de moulage.

Outre les moyens 14 de soufflage, les moyens 12 d'étirage sont également déplacés axialement de la position escamotée vers la position d'utilisation suivant la flèche représentée sur les moyens 12 d'étirage et orientée vers le bas.

Les moyens 12 d'étirage pénètrent alors à l'intérieur de la préforme 30 de stérilisation et entrent en contact avec le fond 36 de celle-ci pour réaliser un étirage axial de ladite préforme 30.

Tel qu'illustré sur la troisième vue (à droite) de la figure pour l'étape (b), les moyens 12 d'étirage poursuivent leur descente à l'intérieur de la cavité 20 de moulage jusqu'à atteindre axialement le fond du moule 16.

Par comparaison avec la première vue, la troisième vue représente le corps creux 50 obtenu à l'issue de l'étape de transformation par étirage-soufflage à partir de la préforme 30 de stérilisation.

Au cours de l'étape (b) de transformation, les moyens 12 d'étirage sont stérilisés par l'agent stérilisant présent initialement à l'intérieur de la préforme 30 de stérilisation.

Avantageusement, au cours de l'étape (b) de transformation, les moyens 12 d'étirage sont maintenus à l'intérieur de la préforme 30 de stérilisation comportant l'agent stérilisant pendant une durée donnée qui est de la durée correspondant à l'étirage d'une préforme de production lors de la fabrication d'un récipient.

Le but de l'étirage de la préforme 30 de stérilisation lors de l'étape (b) est en effet de stériliser les moyens 12 d'étirage en les mettant en contact avec l'agent stérilisant.

Dans le mode de stérilisation de l'installation, la finalité n'est pas de fabriquer un récipient comme lors du mode de production.

C'est la raison pour laquelle, les paramètres concernant les moyens 12 d'étirage tels que la vitesse de déplacement axial, etc. sont déterminés pour obtenir leur stérilisation (et pas nécessairement pour l'obtention d'un récipient).

Avantageusement, les moyens 12 d'étirage sont maintenus à l'intérieur de la préforme 30 de stérilisation comportant l'agent stérilisant puis du corps creux 50 obtenu pendant une durée donnée qui est supérieure ou égale à la durée d'étirage d'une préforme de production pour fabriquer un récipient.

De préférence, la durée donnée de contact des moyens 12 d'étirage avec l'agent stérilisant contenu à l'intérieur de la préforme 30 de stérilisation est obtenue en contrôlant sélectivement les moyens 28 d'actionnement qui commandent en déplacement lesdits moyens 12 d'étirage.

La modification des paramètres de commande des moyens 12 d'étirage sera notamment préférée lorsque les moyens 28 d'actionnement sont constitués par au moins un moteur électrique.

En variante, la durée donnée de contact des moyens 12 d'étirage avec l'agent stérilisant contenu à l'intérieur de la préforme 30 de stérilisation est obtenue en agissant sur la vitesse d'entrainement en rotation du carrousel de la machine de moulage de l'installation de fabrication comportant ledit au moins un dispositif 10 de moulage.

Une telle variante sera notamment préférée lorsque les moyens 28 d'actionnement des moyens 12 d'étirage sont des moyens mécaniques du type à galet et à came.

Au cours de l'étape (b) de transformation, les moyens 12 d'étirage peuvent être déplacés axialement en translation et/ou en rotation, pendant et/ou après la transformation de la préforme 30 de stérilisation en corps creux 50.

Une fois l'étape (b) de transformation de la préforme 30 de stérilisation achevée, les moyens 12 d'étirage sont déplacés axialement par les moyens 28 d'actionnement de la position d'utilisation vers la position escamotée initiale, c'est-à-dire vers le haut tel qu'illustré sur la figure.

Les moyens 14 de soufflage sont également déplacés axialement, relevés de la position basse vers la position haute.

Les moyens 12 d'étirage et les moyens 14 de soufflage occupent alors respectivement la même position que celle illustrée sur la première vue de gauche de l'étape (b).

Le moule 16 du dispositif 10 de moulage en position fermée comporte à l'intérieur de la cavité 20 de moulage le corps creux 50 obtenu à partir de ladite préforme 30 de stérilisation.

Tel qu'illustré sur la figure, le procédé de stérilisation des moyens 12 d'étirage comporte une étape (c) consistant à évacuer hors de l'installation, à l'issue de l'étape de transformation, le corps creux (50) obtenu lors de cette étape à partir de la préforme (30) de stérilisation.

Dans une mise en œuvre, lorsque l'ouverture des moules peut être pilotée indépendamment de leur position angulaire par rapport à un châssis d'une machine (dans le cas de machines rotatives), les demi-moules 18 sont maintenus en position fermée au-delà de leur passage en position d'ouverture lors des phases de production, les moyens 14 de soufflage restant en position basse et les moyens 12 d'étirage restant en position d'utilisation ou déplacés au moins axialement en translation entres lesdites positions escamotée et d'utilisation, voire également en rotation. De ce fait, le temps de contact entre les moyens 12 d'étirage et l'agent stérilisant est notablement augmentée (typiquement sur plus d'un tour de carrousel).

Pour l'étape (c) d'évacuation, on procède à l'ouverture du moule 16 du dispositif 10 de moulage pour extraire ledit corps creux 50 de la cavité 20 de moulage.

De préférence, le corps creux 50 est évacué vers des moyens 52 de collecte tels qu'un bac représenté sur la figure.

L'évacuation du corps creux 50 est avantageusement réalisée directement après son extraction du moule 16 du dispositif 10 de moulage.

Dans une installation comportant une unité de remplissage, l'évacuation des corps creux 50 est réalisée en amont d'une telle unité de remplissage.

Lorsque l'installation comporte des moyens d'obturation, tels qu'un volet coulissant, permettant d'isoler l'unité de remplissage par rapport à la machine de remplissage, lesdits moyens d'obturation sont alors commandés pour occuper leur position de fermeture et l'évacuation a lieu en amont de ce volet.

Le document FR-2.940.964 précité décrit par exemple une installation comportant de tels moyens d'obturation formés par un volet.

Les moyens 52 de collecte sont destinés à récupérer la série de corps creux 50 fabriqués à partir des préformes 30 de stérilisation lors de la mise en œuvre du procédé de stérilisation des moyens 12 d'étirage dans le mode de fonctionnement dit de stérilisation de l'installation.

Avantageusement, ladite série de corps creux 50 évacués de l'installation lors de l'étape (c) est éliminée.

De préférence, la série de corps creux 50 est éliminée par broyage pour être transformée en un broyat de matière thermoplastique.

Avantageusement, le broyat obtenu est traité de manière à éliminer l'agent stérilisant susceptible d'être présent et permettre un recyclage de la matière thermoplastique.

La matière thermoplastique est par exemple recyclée pour fabriquer des préformes par moulage par injection ou par extrusion.

## Revendications

1. Procédé pour la stérilisation des moyens (12) d'étirage d'un dispositif (10) de moulage de récipients équipant une installation de fabrication de récipients en matière thermoplastique obtenus par étirage-soufflage à partir de préformes de production, ledit procédé de stérilisation des moyens (12) d'étirage comporte au moins les étapes consistant à :
- introduire une quantité déterminée d'un agent stérilisant à l'intérieur d'une préforme (30) de stérilisation,
- au cours d'une étape dite de transformation, transformer ladite préforme (30) de stérilisation en corps creux (50) par étirage-soufflage dans ledit dispositif (10) de moulage,
**caractérisée en ce que** ladite introduction de l'agent stérilisant se fait à l'état de vapeur dans une préforme (30) de stérilisation ayant une température permettant d'obtenir un dépôt par condensation d'un film de buée uniforme sur une surface (32) interne de ladite préforme (30) de stérilisation, et il est procédé à la transformation successive en corps creux (50) d'au moins deux préformes (30) de stérilisation dans un même dispositif (10) de moulage, et
dans lequel l'étape de transformation consiste à obtenir un récipient partiellement formé.

2. Procédé selon la revendication 1, dans lequel l'agent stérilisant contient du peroxyde d'hydrogène, le procédé comprenant une étape d'activation du peroxyde d'hydrogène de façon à former des radicaux libres, ladite activation de l'agent stérilisant de chaque préforme (30) de stérilisation ayant lieu avant et/ou pendant l'étape de transformation de ladite préforme (30) de stérilisation en corps creux (50) par étirage-soufflage.

3. Procédé selon la revendication 1 ou 2, comprenant une étape de conditionnement thermique des préformes de production et des préformes (30) de stérilisation, l'étape d'introduction d'une quantité d'agent stérilisant se faisant avant et/ou pendant l'étape de conditionnement thermiques des préformes de stérilisations

4. Procédé selon l'une des revendications précédentes, comprenant une étape d'évacuer hors de l'installation, à l'issue de l'étape de transformation, les corps creux (50) obtenus à partir des préformes (30) de stérilisation.

5. Procédé selon l'une des revendications précédentes, **caractérisé en ce que** lesdites étapes du procédé de stérilisation des moyens (12) d'étirage sont mises en œuvre dans un mode de fonctionnement de l'installation, dit mode de stérilisation, dans lequel le dispositif (10) de moulage fabrique une série de corps creux (50).

6. Procédé selon l'une des revendications précédentes, **caractérisé en ce que** le procédé utilise ladite installation présentant un mode de fonctionnement de production au cours duquel des récipients sont formés à partir de préformes de production sensiblement identiques audites préformes de stérilisation, et au cours duquel un agent de stérilisation de production est préalablement introduit dans la préforme de production.

7. Procédé selon la revendication 6, **caractérisé en ce qu'**au cours du mode de fonctionnement de production, les moyens (12) d'étirage de l'installation sont maintenus à l'intérieur de la préforme (30) de production pendant une durée donnée
et en ce au cours dudit procédé de stérilisation des moyens d'étirage, les moyens (12) d'étirage sont maintenus à l'intérieur de la préforme (30) de stérilisation comportant l'agent stérilisant pendant une autre durée donnée qui est supérieure à la durée donnée pour le mode de production.

8. Procédé selon la revendication 7, **caractérisé en ce que** ladite autre durée donnée de contact des moyens (12) d'étirage avec l'agent stérilisant contenu à l'intérieur de la préforme (30) de stérilisation est obtenue :
en contrôlant sélectivement des moyens (28) d'actionnement qui commandent en déplacement lesdits moyens (12) d'étirage,
et/ou en agissant sur une vitesse d'entrainement en rotation d'un carrousel d'une machine de moulage de l'installation de fabrication comportant au moins le dispositif (10) de moulage de récipients.

9. Procédé selon l'une des revendications 6 à 8, **caractérisé en ce qu'**au cours dudit procédé de stérilisation, ledit agent stérilisant introduit dans une préforme (30) de stérilisation est le même agent de stérilisation de production.

10. Procédé selon l'une des revendications 6 à 9, **caractérisé en ce qu'**au cours du mode de fonctionnement de production, la quantité d'agent stérilisant introduite dans la préforme (30) de production est une quantité donnée
et **en ce qu'**au cours dudit procédé de stérilisation des moyens d'étirage, la quantité d'agent stérilisant introduite dans la préforme (30) de stérilisation est supérieure à la quantité donnée pour le mode de production.

11. Procédé selon l'une des revendications 6 à 10, **caractérisé en ce qu'**au cours dudit procédé de stérilisation ledit agent stérilisant introduit dans la préforme (30) de stérilisation présente des paramètres physico-chimiques différents des paramètres physico-chimiques de l'agent de stérilisation de production, et en particulier présente une concentration supérieure à celui introduit dans une préforme de production.

12. Procédé selon l'une quelconque des revendications 6 à 11, caractérisé en ce en ce qu'au cours du mode de fonctionnement de production, les préformes (30) de production sont chauffées à une température donnée pour le mode de production
et en ce qu'au cours dudit procédé de stérilisation des moyens d'étirage, la préforme (30) de stérilisation est chauffée, avant l'étape de transformation en corps creux (50), à une température donnée qui est différente de la température donnée pour le mode de production.

13. Procédé selon l'une quelconque des revendications 6 à 12, **caractérisé en ce qu'**au cours du mode de fonctionnement de production, les préformes (30) de production sont étendues jusqu'à former ledit récipient totalement formé au moyen d'un fluide de soufflage présentant une pression maximale pour le mode de production dudit récipient
et **en ce qu'**au cours dudit procédé de stérilisation des moyens d'étirage, la préforme (30) de stérilisation est transformée en corps creux (50) par étirage-soufflage au moyen d'un fluide de soufflage présentant une pression maximale qui est inférieure à ladite pression maximale pour le mode de production.

14. Procédé selon l'une quelconque des revendications 6 à 13, **caractérisé en ce que** l'installation utilisée par le procédé est une machine rotative comprenant une pluralité de dispositifs de moulage équipés chacun de demi-moules (18), **en ce qu'au** cours du mode de fonctionnement de production, les demi-moules s'ouvrent dans une position angulaire d'ouverture, **et en ce qu'au** cours dudit procédé de stérilisation des moyens d'étirage, les demi-moules sont maintenus en position fermée au-delà de leurs passage dans la position angulaire d'ouverture.

## Patentansprüche

1. Verfahren zur Sterilisation der Streckmittel (12) einer Behälterformvorrichtung (10), mit der eine Anlage zur Fertigung von Behältern aus thermoplastischem Material ausgestattet ist, die durch Streckblasen aus Produktions-Preformen erhalten werden, wobei das Verfahren zur Sterilisation der Streckmittel (12) wenigstens die folgenden Schritte umfasst:
- Einführen einer bestimmten Menge eines Sterilisationsmittels ins Innere einer Sterilisations-Preform (30),
- im Verlaufe eines sogenannten Umformungsschrittes, Umformen der Sterilisations-Preform (30) in einen Hohlkörper (50) durch Streckblasen in der Formvorrichtung (10),
**dadurch gekennzeichnet, dass** die Einführung des Sterilisationsmittels im dampfförmigen Zustand in eine Sterilisations-Preform (30) erfolgt, die eine Temperatur aufweist, welche ermöglicht, eine Kondensationsabscheidung eines gleichmäßigen Niederschlagsfilms auf einer Innenfläche (32) der Sterilisations-Preform (30) zu bewirken, und dass nacheinander die Umformung von wenigstens zwei Sterilisations-Preformen (30) in Hohlkörper (50) in derselben Formvorrichtung (10) vorgenommen wird, und
wobei der Umformungsschritt darin besteht, einen teilweise geformten Behälter zu erhalten.

2. Verfahren nach Anspruch 1, wobei das Sterilisationsmittel Wasserstoffperoxid enthält, wobei das Verfahren einen Schritt der Aktivierung des Wasserstoffperoxids umfasst, um freie Radikale zu bilden, wobei die Aktivierung des Sterilisationsmittels jeder Sterilisations-Preform (30) vor und/oder während des Schrittes der Umformung der Sterilisations-Preform (30) in einen Hohlkörper (50) durch Streckblasen erfolgt.

3. Verfahren nach Anspruch 1 oder 2, welches einen Schritt der thermischen Konditionierung der Produktions-Preformen und der Sterilisations-Preformen (30) umfasst, wobei der Schritt der Einführung einer Menge eines Sterilisationsmittels vor und/oder während des Schrittes der thermischen Konditionierung der Sterilisations-Preformen durchgeführt wird.

4. Verfahren nach einem der vorhergehenden Ansprüche, welches nach Beendigung des Umformungsschrittes einen Schritt des Entnehmens der aus den Sterilisations-Preformen (30) erhaltenen Hohlkörper (50) aus der Anlage umfasst.

5. Verfahren nach einem der vorhergehenden Ansprüche, **dadurch gekennzeichnet, dass** die Schritte des Verfahrens zur Sterilisation der Streckmittel (12) in einer "Sterilisationsmodus" genannten Betriebsart der Anlage durchgeführt werden, in welcher die Formvorrichtung (10) eine Reihe von Hohlkörpern (50) fertigt.

6. Verfahren nach einem der vorhergehenden Ansprüche, **dadurch gekennzeichnet, dass** das Verfahren die Anlage verwendet, die eine Produktions-Betriebsart aufweist, in welcher Behälter aus Produktions-Preformen geformt werden, die im Wesentlichen mit den Sterilisations-Preformen identisch sind, und in welcher ein Produktions-Sterilisationsmittel zuvor in die Produktions-Preform eingeführt wird.

7. Verfahren nach Anspruch 6, **dadurch gekennzeichnet, dass** in der Produktions-Betriebsart die Streckmittel (12) der Anlage während einer vorgegebenen Dauer im Inneren der Produktions-Preform (30) gehalten werden,
und dadurch, dass bei dem Verfahren zur Sterilisation der Streckmittel die Streckmittel (12) im Inneren der Sterilisations-Preform (30) gehalten werden, die das Sterilisationsmittel während einer weiteren vorgegebenen Dauer aufweist, welche länger als die für den Produktionsmodus vorgegebene Dauer ist.

8. Verfahren nach Anspruch 7, **dadurch gekennzeichnet, dass** die weitere vorgegebene Dauer des Kontakts der Streckmittel (12) mit dem im Inneren der Sterilisations-Preform (30) enthaltenen Sterilisationsmittel erhalten wird:
durch selektives Steuern von Betätigungsmitteln (28), welche die Bewegung der Streckmittel (12) steuern,
und/oder durch Ändern einer Drehantriebsgeschwindigkeit eines Karussells einer Formmaschine der Fertigungsanlage, die wenigstens eine Behälterformvorrichtung (10) aufweist.

9. Verfahren nach einem der Ansprüche 6 bis 8, **dadurch gekennzeichnet, dass** bei dem Verfahren zur Sterilisation das Sterilisationsmittel, das in die Sterilisations-Preform (30) eingeführt wird, dasselbe wie das Produktions-Sterilisationsmittel ist.

10. Verfahren nach einem der Ansprüche 6 bis 9, **dadurch gekennzeichnet, dass** in der Produktions-Betriebsart die Menge an Sterilisationsmittel, die in die Produktions-Preform (30) eingeführt wird, eine vorgegebene Menge ist,
und dadurch, dass bei dem Verfahren zur Sterilisation der Streckmittel die Menge an Sterilisationsmittel, die in die Sterilisations-Preform (30) eingeführt wird, größer als die für den Produktionsmodus vorgegebene Menge ist.

11. Verfahren nach einem der Ansprüche 6 bis 10, **dadurch gekennzeichnet, dass** bei dem Verfahren zur Sterilisation das Sterilisationsmittel, das in die Sterilisations-Preform (30) eingeführt wird, physikalisch-chemische Parameter aufweist, die von den physikalisch-chemischen Parametern des Produktions-Sterilisationsmittels verschieden sind, und insbesondere eine Konzentration aufweist, die höher als diejenige ist, die in eine Produktions-Preform eingeführt wird.

12. Verfahren nach einem der Ansprüche 6 bis 11, **dadurch gekennzeichnet, dass** in der Produktions-Betriebsart die Produktions-Preformen (30) auf eine vorgegebene Temperatur für den Produktionsmodus erwärmt werden,
und dadurch, dass bei dem Verfahren zur Sterilisation der Streckmittel die Sterilisations-Preform (30) vor dem Schritt der Umformung in einen Hohlkörper (50) auf eine vorgegebene Temperatur erwärmt wird, welche von der vorgegebenen Temperatur für den Produktionsmodus verschieden ist.

13. Verfahren nach einem der Ansprüche 6 bis 12, **dadurch gekennzeichnet, dass** in der Produktions-Betriebsart die Produktions-Preformen (30) mittels eines Blasfluids, das einen maximalen Druck für den Produktionsmodus des Behälters aufweist, erweitert werden, bis der vollständig ausgebildete Behälter geformt ist,
und dadurch, dass bei dem Verfahren zur Sterilisation der Streckmittel die Sterilisations-Preform (30) durch Streckblasen mittels eines Blasfluids in einen Hohlkörper (50) umgeformt wird, das einen maximalen Druck aufweist, welcher niedriger als der maximale Druck für den Produktionsmodus ist.

14. Verfahren nach einem der Ansprüche 6 bis 13, **dadurch gekennzeichnet, dass** die bei dem Verfahren verwendete Anlage eine Rotationsmaschine ist, die mehrere Formvorrichtungen umfasst, die jeweils mit Formhälften (18) ausgestattet sind, dadurch, dass in der Produktions-Betriebsart die Formhälften sich in eine Öffnungs-Winkelposition öffnen, und dadurch, dass bei dem Verfahren zur Sterilisation der Streckmittel die Formhälften über ihren Übergang in die Öffnungs-Winkelposition hinaus in einer geschlossenen Position gehalten werden.

## Claims

1. Method for sterilizing stretching means (12) of a container moulding device (10) equipping an installation for manufacturing containers made of thermoplastic material obtained by stretch-blow moulding from production preforms, said method for sterilizing stretching means (12) comprises at least the steps consisting in:
- introducing a determined quantity of a sterilizing agent inside a sterilization preform (30),
- during a so-called transformation step, transforming said sterilization preform (30) into a hollow body (50) by stretch-blow moulding in said moulding device (10),
**characterized in that** said introduction of the sterilizing agent is done in the vapour state in a sterilization preform (30) having a temperature allowing a deposition to be obtained by condensation of a uniform film of mist on an internal surface (32) of said sterilization preform (30), and the successive transformation into hollow bodies (50) of at least two sterilization preforms (30) in one and the same moulding device (10) is carried out, and
in which the transformation step consisting in obtaining a partially formed container.

2. Method according to Claim 1, wherein the sterilizing agent contains hydrogen peroxide, the method comprising a step of activation of the hydrogen peroxide so as to form free radicals, said activation of the sterilizing agent of each sterilization preform (30) taking place before and/or during the step of transformation of said sterilization preform (30) into a hollow body (50) by stretch-blow moulding.

3. Method according to Claim 1 or 2, comprising a step of thermal conditioning of the production preforms and of the sterilization preforms (30), the step of introduction of a quantity of sterilizing agent being done before and/or during the step of thermal conditioning of the sterilization preforms.

4. Method according to one of the preceding claims, comprising a step of discharging, from the installation at the end of the transformation step, the hollow bodies (50) obtained from the sterilization preforms (30).

5. Method according to one of the preceding claims, **characterized in that** said steps of the method for sterilizing stretching means (12) are implemented in a mode of operation of the installation, called sterilization mode, in which the moulding device (10) manufactures a series of hollow bodies (50).

6. Method according to one of the preceding claims, **characterized in that** the method uses said installation having a production operating mode during which containers are formed from production preforms substantially identical to said sterilization preforms, and during which a production sterilization agent is previously introduced into the production preform.

7. Method according to Claim 6, **characterized in that**, during the production operating mode, the stretching means (12) of the installation are maintained inside the production preform (30) for a given duration and **in that**, during said method for sterilizing stretching means, the stretching means (12) are maintained inside the sterilization preform (30) comprising the sterilizing agent for another given duration which is greater than the given duration for the production mode.

8. Method according to Claim 7, **characterized in that** said other given duration of contact of the stretching means (12) with the sterilizing agent contained inside the sterilization preform (30) is obtained:
by selectively controlling actuation means (28) which control the displacement of said stretching means (12),
and/or by acting on a rotational driving speed of a carousel of a moulding machine of the manufacturing installation comprising at least the container moulding device (10).

9. Method according to one of Claims 6 to 8, **characterized in that**, during said sterilization method, said sterilizing agent introduced into a sterilization preform (30) is the same production sterilization agent.

10. Method according to one of Claims 6 to 9, **characterized in that**, during the production operating mode, the quantity of sterilizing agent introduced into the production preform (30) is a given quantity
and **in that**, during said method for sterilizing the stretching means, the quantity of sterilizing agent introduced into the sterilization preform (30) is greater than the given quantity for the production mode.

11. Method according to one of Claims 6 to 10, **characterized in that**, during said sterilization method, said sterilizing agent introduced into the sterilization preform (30) has physico-chemical parameters that are different from the physico-chemical parameters of the production sterilization agent, and in particular has a concentration greater than that introduced into a production preform.

12. Method according to any one of Claims 6 to 11, **characterized in that**, during the production operating mode, the production preforms (30) are heated to a given temperature for the production mode
and **in that**, during said method for sterilizing the stretching means, the sterilization preform (30) is heated, before the step of transformation into a hollow body (50), to a given temperature which is different from the given temperature for the production mode.

13. Method according to any one of Claims 6 to 12, **characterized in that**, during the production operating mode, the production preforms (30) are extended to form said totally formed container by means of a blowing fluid having a maximum pressure for the production mode for said container and **in that**, during said method for sterilizing the stretching means, the sterilization preform (30) is transformed into a hollow body (50) by stretch-blow moulding by means of a blowing fluid having a maximum pressure which is lower than said maximum pressure for the production mode.

14. Method according to any one of Claims 6 to 13, **characterized in that** the installation used for the method is a rotary machine comprising a plurality of moulding devices each equipped with half-moulds (18), **in that**, during the production operating mode, the half-moulds are opened to an angular opening position, and **in that**, during said method for sterilizing the stretching means, the half-moulds are maintained in the closed position beyond their transition to the angular opening position.
